# EUROPEAN PATENT SPECIFICATION

(11) **EP 0 329 489 B1**
(45) Date of publication and mention of the grant of the patent: **07.12.1994**
(21) Application number: 89301625.3
(22) Date of filing: 20.02.1989
(51) Int. Cl.: A61B 5/02, A61B 8/06

(54) **Improvements in or relating to medical apparatus and procedures**
Medizinische Geräte und Verfahren
Appareils médicaux et procédés

(30) Priority: 18.02.1988 GB 8803840
(43) Date of publication of application: 23.08.1989
(73) Proprietor: VASCULAR SURGICAL FORUM, Bristol BS2 8HW (GB)
(72) Inventor: Beard, Jonathan David Department of Surgery, Leicester LE2 7LX (GB)
(74) Representative: Abrams, Michael John

(56) References cited:
- EP-A- 0 016 399
- EP-A- 0 041 696
- US-A- 4 425 922

## Description

This invention relates to medical apparatus and surgical procedures which are of particular value in connection with femorodistal by-pass graft procedures.

The decision to attempt a femorodistal bypass graft is usually based on the pre-operative demonstration of an adequate calf vessel runoff. It is an advantage to know the state of the runoff vessels as this may affect the level of the distal anastomosis and influence graft patency. When the popliteal artery is occluded it is essential to detect which calf vessel, if any, is patent as this will determine whether grafting is at all possible. Assessment of the pedal arch and its calf vessel connections may also influence the site of the distal anastomosis.

The recent upsurge in popularity of femorodistal grafts for limb salvage has highlighted the shortcomings of conventional pre-operative arteriography. About one-quarter of crural or pedal arteries that are judged patent on Doppler ultrasonography or direct operative exploration may fail to opacify. The use of specialised techniques such as reactive hyperaemia, vasodilators, intra-operative arteriography, or digital subtraction arteriography may increase the visualization of distal vessels but they are not always practicable or available.

Conventional pre-operative arteriography may fail to demonstrate patent calf and foot vessels, especially in the presence of severe ischaemia. Although distal vessels should be judged occluded only if there is filling of the small collaterals, this may not be achieved on the initial arteriograms. It may be uncertain whether failure to demonstrate distal vessels is due to technical factors or due to occlusion. Repeat arteriograms incur additional expense and delay with no guarantee of improved definition. Worse still, it may be assumed that vessels are occluded simply because they have not been filled with contrast medium, and the patient may thus be denied the chance of reconstruction.

Doppler ultrasonography may detect patent crural or pedal vessels that are missed on arteriography but may itself miss patent vessels because of severely damped signals due to a low perfusion pressure. Diligent searching over the area of the vessel without pressure from the probe on the skin and increasing the perfusion pressure by making the feet dependent may help, but require patience and expertise.

An ideal system for assessing distal vessel patency would be non-invasive, safe, simple and rapid to perform. Standard Doppler ultrasound is useful but may also miss patent vessels if signals are severely damped despite a meticulous technique.

A non-invasive method which may be used, inter alia, in determining calf vessel patency has now been developed. More particularly, according to one aspect of the present invention, there is provided a method of testing the vascular condition of a human or a non-human animal, the method being characterised by the steps of:
i) selecting a portion of the body of the animal which has restricted blood flow due to an ischaemia;
ii) applying a pulsatile pressure waveform as a series of pressure pulses to a first part of said portion in order to generate a pulsatile pressure waveform in the blood in said portion, and producing by way of means a first signal representative of said applied pulsatile pressure waveform;
iii) non-invasively detecting the pulsatile pressure waveform in the blood at a second part of said portion spaced from said first part, and producing a second signal representative of the detected pulsatile pressure waveform; and
iv) comparing the waveform of the first signal with the waveform of the second signal.

This novel method can be applied so as to generate blood flow in patent calf arteries by means of a pulsatile cuff located about the calf of a patient.

Preferably, the detection of the pulsatile pressure waveform in the blood is at a point distal with respect to the location of the point of application of said pressure pulses and is effected by means of a Doppler velocimeter. Preferably, the pressure pulses are applied via a cuff which is positioned round the patient's calf. The pressure pulses are advantageously produced pneumatically. Preferably the pulsatile pressure waveform has a pulse rate in the range 0.3 - 1.5 pulses per second. A pulse rate of 0.5-1 pulse/second is expected to be satisfactory in most cases. The magnitude of the pressure pulse preferably is in the range (19-66.5) x 10³ Nm⁻² (150-500 mm Hg). The most advantageous pressure range is (26-40) x 10³ Nm⁻² (200-300 mm Hg). The applied pressure may persist for the order of a few milliseconds before it decays to normal pressure. Such an arrangement can conveniently be achieved by using a supply of compressed gas connected to a control arrangement, e.g. a solid state pressure transducer which is used to actuate a three-way valve; one arm of the valve is connected to the source of compressed gas, the second arm is connected to the cuff, and the third arm vents to the atmosphere. The solid state pressure transducer admits gas from the source to the cuff until the pressure reaches a predetermined level, at which point the first arm (to the gas source) is closed and the third arm (which vents to the atmosphere) is put into communication with the cuff. When the pressure transducer senses that the cuff pressure has reduced to zero, it closes the vent arm and re-admits compressed gas to the cuff.

U.S. 4,425,922 discloses a non-invasive method and apparatus for detecting the presence of abnormal blood flow and which involves producing two signals from measurements on a body at spaced parts of the body and the comparison of the waveforms of the signals. However, even though the document discloses the additional use of an inflatable cuff to restrict blood flow, the signals compared do not relate to pulsatile pressure waveforms in body zones which are ischaemic, but rather in one case to an ECG and in the other to blood streaming potential.

According to a second aspect of the present invention, there is provided non-invasive apparatus for use in testing the condition of blood vessels downstream from an ischaemia in a human or non-human animal, which apparatus is characterised in that there is provided:
i) means for producing blood flow having a pulsatile waveform downstream of the ischaemia by applying to the periphery of a first part of the body of said animal a pulsatile pressure waveform, and for generating a first signal representative of said applied pulsatile waveform;
ii)means for detecting the waveform of the blood flow at a second part of the body of said animal distal to said first part, and for generating a second signal representative of said detected waveform; and
iii) means enabling a comparison to be made between the waveform of said first signal and the waveform of said second signal.

Conveniently, the pulsatile pressure waveform producing means comprise (1) a source of compressed gas; (2) a pressure control system; and (3) an inflatable cuff - shaped so as to be locatable about that area of a patient (e.g. his calf) which is to be investigated. The pressure control system is preferably in the form of a pressure transducer which actuates a three-way valve, whereby compressed gas is admitted to the inflatable cuff until a predetermined pressure cut-off point is reached, whereupon the pressure transducer stops the supply of compressed gas and vents the interior of the inflatable cuff to the atmosphere; when the pressure within the inflatable cuff falls to zero (gauge), the pressure transducer closes the vent port and re-admits compressed gas to the inflatable cuff. This cycle is repeated generating thereby the pulsatile pressure wave form.

Blood flow at the distal location is preferably detected by a Doppler ultrasound technique. A conventional Doppler velocimeter (e.g. operating in the range 2 to 12 MHz) may be used for this purpose.

The apparatus and techniques described above are particularly valuable in assessing whether or not to perform a femorodistal bypass graft.

For a better understanding of the invention, and to show how the same may be carried into effect, reference will now be made, by way of example, to the accompanying drawings, in which:
FIGURE 1 illustrates the application of the invention to assessment of calf vessel patency;
FIGURE 2 illustrates the pulsaltile blood flow as observed by a Doppler velocimeter in the course of an assessment using the apparatus and techniques of this invention;
FIGURE 3 is a plot showing the correlation between calf vessel patency as determined in accordance with the present invention on the one hand and as determined by arteriography on the other hand;
FIGURE 4 is a plot of the peripheral resistance of calf vessels against the assessment of calf vessel patency as determined using apparatus and techniques in accordance with the invention;
FIGURE 5 is a diagram showing the pneumatic circuitry in one embodiment of this invention; and
FIGURE 6 is a logic circuit corresponding to the pneumatic circuitry of Figure 5.

In one preferred embodiment, the present invention uses a standard 10 MHz Doppler ultrasound velocimeter and a sphygmomanometer cuff driven by pulsatile compressed air. The cuff is placed around the upper calf and the pulsatile pressure generates blood flow in the calf arteries. Patent arteries can be detected by the Doppler probe at the ankle even if the existing signal is inaudible. The pedal arch patency test is also easily performed to determine continuity with the pedal arch. An occlusive thigh cuff is occasionally required to prevent interference by the normal arterial signal although it is not usually necessary in severely ischaemic limbs. Venous signals are readily differentiated from arterial ones as they are characteristically attenuated by the venous valves which prevented retrograde flow. Theoretically there might be a problem in patients with deep venous incompetence but this has not been encountered clinically.

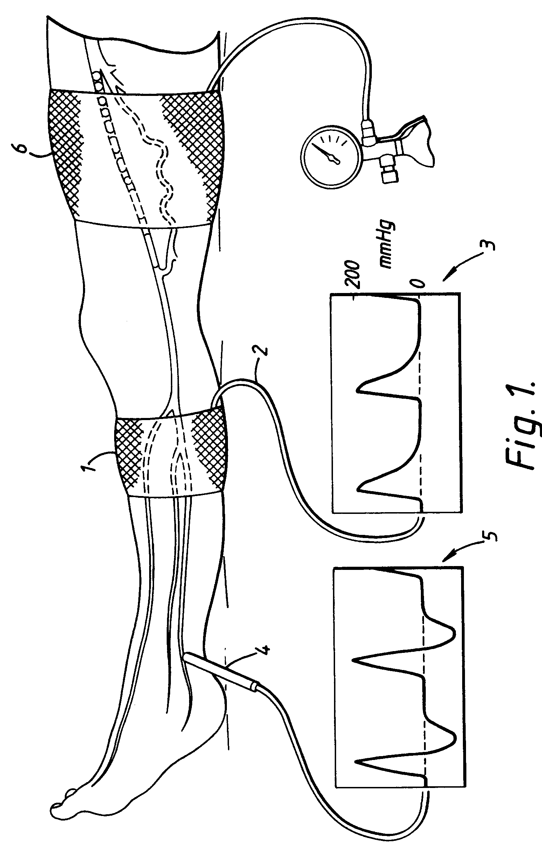

Referring to Figure 1, there is shown schematically the arrangement adopted in utilising the PGR system described above. An inflatable cuff 1 is positioned around the patient's calf. A pulsatile pressure waveform is applied by the cuff which is fed with compressed air via a control arrangement (not shown) and supply tube 2. The pressure waveform is depicted schematically at 3, Blood flow in the runoff vessels is determined using a conventional Doppler velocimeter 4 which is held in contact with the patient's limb in the ankle region in order to detect blood flow in one of the three runoff arteries. The output of the Doppler velocimeter is illustrated at 5 (showing the signal obtained where runoff is adequate). A further cuff 6 is shown located about the patient's thigh; this functions as a conventional sphygmomanometer cuff and is required only where the normal arterial signal would interfere with that deriving from the PGR system - i.e. in patients with relatively little ischaemia.

The control unit is driven by compressed air and consists of two separate modules controlling the pulsatile and occlusive cuffs. The inflation and deflation of the pulsatile cuff is controlled by a two-way solenoid valve and an in-line solid-state pressure transducer. The pulse pressure can be varied from (0-40) x 10³ Nm⁻² (0 to 300 mm Hg) and the frequency of pulsation from 0 to 100/min but a standard pulse of 33 x 10³ Nm⁻² (250 mm Hg) at a rate of 50/min is usually used. The occlusive cuff pressure is controlled by a standard pressure regulator and can be varied from (0-40) x 10³ Nm⁻² (0 to 300 mm Hg). For convenience, the system just described above is termed herein "pulse generated runoff" or "PGR".

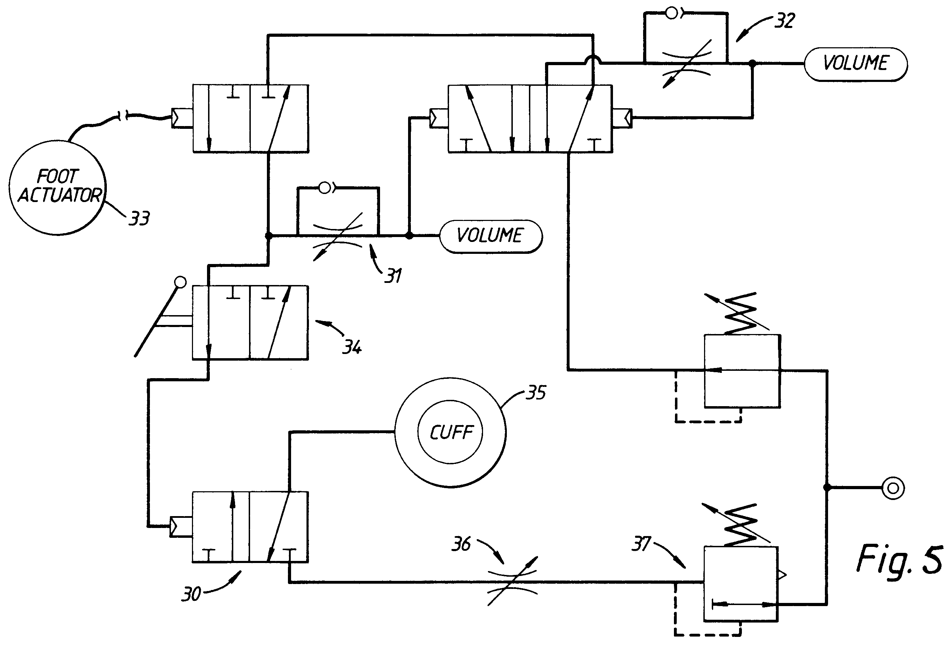

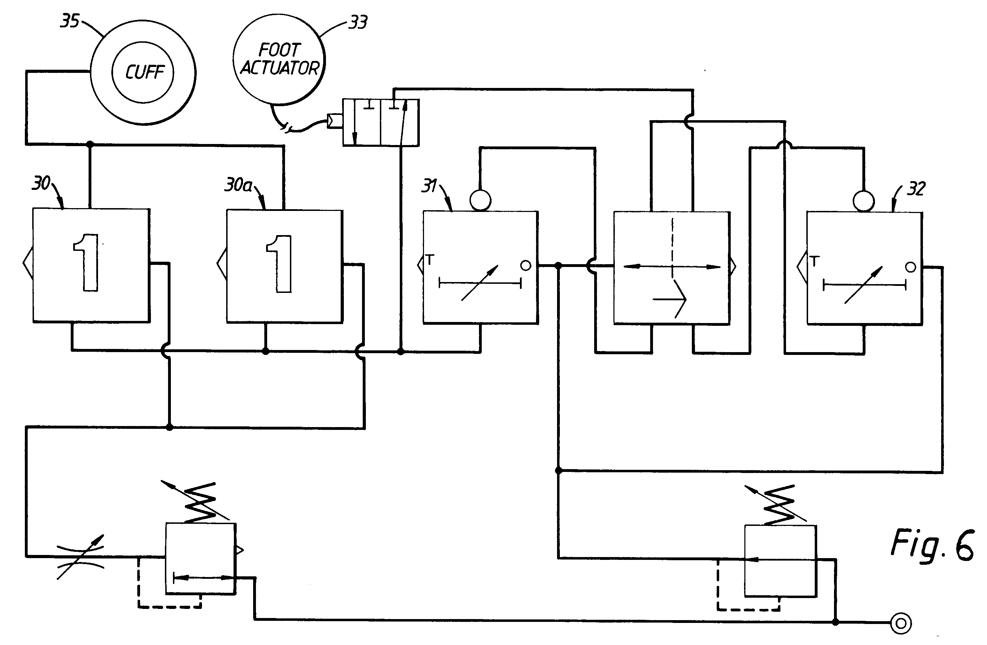

Figures 5 and 6 illustrate circuitry used in one embodiment of apparatus in accordance with this invention. Conventional symbols are used in the two circuits to denote conventional components as will be recognised by those skilled in the art of pneumatics. The upper part of the circuit of Figure 5 functions as a timer circuit which produces a pulse to a cuff inflate/deflate valve 30. The time in which the cuff is inflated and deflated can be adjusted by means of two throttle valves 31 and 32. The system is activated by a foot actuator 33 connected into the timer circuit in such a way that it can stop a pressure pulse activating the cuff inflate/deflate valve 30. The pressure pulse is fed to valve 30 via a two way toggle valve 34 which acts as an emergency deflate valve. Valve 30 is connected to the cuff 35 for inflation/deflation thereof. Deflation of the cuff takes place straight through valve 30 to exhaust without any throttle since it is desirable for deflation to occur as rapidly as possible. The remainder of the pneumatic circuit comprises a throttle 36 and a cuff supply regulator 37.

The logic circuit of Figure 6 corresponds to the pneumatic circuit of Figure 5 except that there is no emergency deflate valve 34, and there are two inflate/deflate valves 30, 30a in order to provide an increased volume of air to the cuff 35.

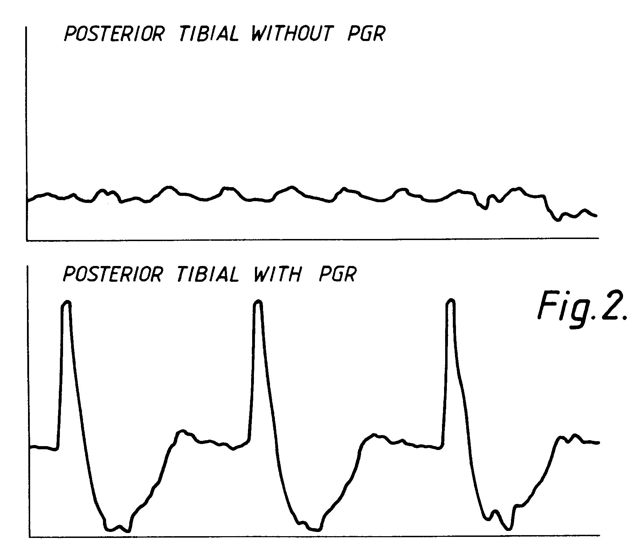

Using the PGR system a search was made in a series of human patients for all three calf vessels at the level of the ankle (anterior tibial, posterior tibial and peroneal arteries). Each vessel was scored 2 for a normal biphasic signal, 1 for a damped monophasic signal and 0 for no signal, giving a possible total of 0-6. A normal signal has a sharp upstroke as the cuff inflates, an amplitude >2cm and reversed flow as the cuff deflates (Figure 2).

Pre-operative arteriograms also carried out on the same group of patients, and were were scored at a weekly meeting of the consultant vascular surgeons and radiologists. Each calf vessel was scored 2 if patent to the ankle, 1 if patent but diseased and 0 if occluded, again giving a possible total of 0-6. Arteriograms were judged to be inadequate if there was no filling of calf collaterals on any of the series. Assessment of the plantar arch proved to be impossible because it was rarely adequately demonstrated.

Ninety-five ischaemic limbs with superficial femoral artery occlusion were studied in 76 patients (49 men and 27 women, aged 42-92 years, median 76 years). Of these limbs, 68 were critically ischaemic with rest pain and/or gangrene and 27 had symptoms of claudication only. All patients underwent transfemoral aortography except for six who had unilateral femoral arteriograms. Pulse-generated runoff (PGR) assessments were made on all limbs within 24 h of arteriography.

Ten control limbs were studied in five patients with isolated aortoiliac disease, whose arteriograms demonstrated three patent calf vessels down to the ankle.

The peripheral resistance was measured in all of the 62 limbs undergoing femorodistal reconstruction or amputation. Of the 62 limbs, 9 received a primary amputation based upon the pre-operative arteriogram and the degree of tissue loss. The remaining re-underwent exploration with a view to reconstruction, and of these, 5 received a below-knee amputation, the other 48 receiving an in situ femorodistal vein graft. The decision to amputate was made by the consultant surgeon if the distal vessels were occluded or severely diseased. The distal anastomosis was to the infrageniculate popliteal in 29 limbs, the tibioperoneal trunk in 10 and to a calf vessel in 9 limbs.

The peripheral resistance was measured by a method similar to that described by Parvin (Br J Surg 1985; 72; 751-3). An infusion of heparinised blood was made by hand, via a soft 6 or 8 French PVC catheter inserted through an arteriotomy into the vessel chosen for the distal anastomosis. The resistance was calculated from the simultaneously recorded pressure and flow, a deduction being made for the resistance of the catheter. The resistance in the primary amputation group was measured in the calf vessel thought best on exploration before amputation. The results obtained are described below.

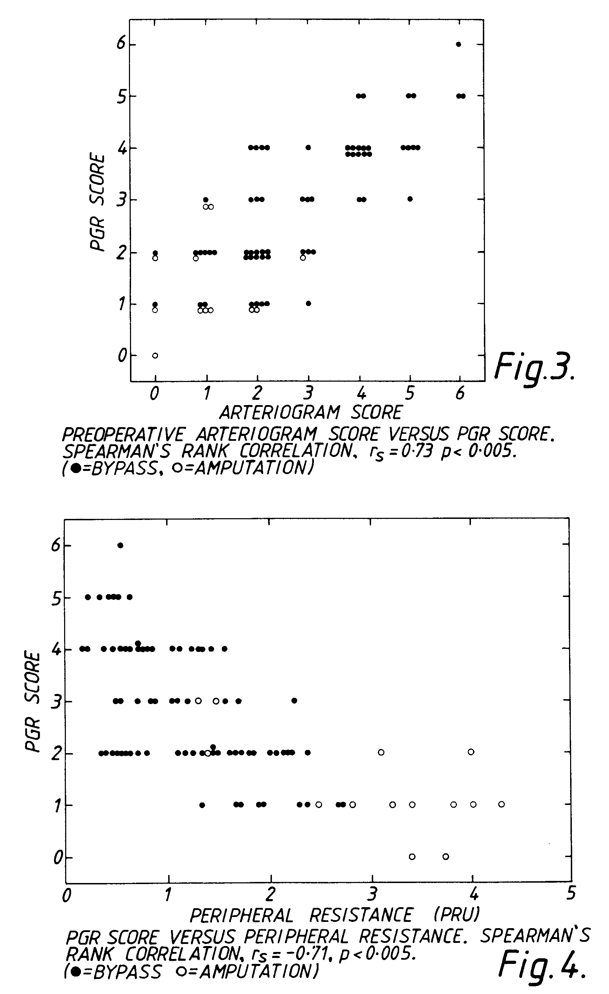

The 10 control limbs with arteriogram scores of 6 all scored 6 on the PGR assessment. Seventeen arteriograms (18 per cent) were judged inadequate for scoring leaving seventy-eight for comparison with the PGR (Figure 3). There was a highly significant correlation between the arteriogram and the PGR scores (Spearman's Rank Correlation, 0.74; P<0.001). In severely ischaemic limbs the PGR tended to detect more vessels than arteriography, detecting at least one patent vessel in eight limbs (8 per cent) where no vessel was judged patent and in sixteen limbs (17 per cent) where the arteriogram was judged inadequate.

The peripheral resistance, measured in the 62 operated limbs, correlated better with the PGR than the arteriogram score (Figure 4) although both were highly significant (Spearman's Rank Correlation, -0.71 and -0.54 respectively; P<0.001). There was a highly significant difference between the peripheral resistance of amputated limbs and those undergoing femorodistal bypass (Mann-Whitney U test, P=0.0001). Three of the primary amputations had resistances which would have been compatible with reconstruction. No vessels were detected by pre-operative arteriography or conventional Doppler examination but the PGR system demonstrated at least one patent vessel in all three of these limbs.

It was initially thought that the PGR system worked by direct compression of the popliteal and calf arteries. However, satisfactory signals were obtained on diabetic limbs with heavily calcified incompressible vessels. This suggests that compression of the calf muscles was the source, the blood flowing retrogradely into the calf arteries. Patients did not find the pulsatile cuff unduly uncomfortable and no problems of increased ischaemia due to arterial damage were encountered.

The use of the pulse-generated runoff (PGR) system in accordance with this invention means that the distal vessels can be assessed independently of any proximal disease. Detection of patent vessels in the calf or foot is quickly and easily performed because of the enhanced flow signals.

The PGR system described above is inexpensive to construct and can be used with any available Doppler velocimeter. PGR correlates significantly with the pre-operative arteriogram score but in severely ischaemic limbs may detect up to 25 per cent more patent vessels. There is a better correlation between the peripheral resistance and PGR than the arteriogram score, suggesting that PGR is a more physiological test of runoff. Pre-operative arteriography usually shows the popliteal and upper calf vessels sufficiently well to indicate the necessary level of bypass. PGR is able to confirm the patency of the distal calf and foot vessels and helps to determine the best vessel for exploration.

Although described above in relation to the assessment of arterial patency in the calf, the present invention may also be used inter alia to assess the condition of veins. For example, in healthy veins, the venous valves will prevent retrograde blood flow; hence if a Doppler velocimeter is used to assess venous flow in the technique described above, the Doppler signal will indicate whether or not there is deep venous incompetence. This condition will allow retrograde blood flow and thus will give a Doppler signal somewhat resembling that obtained with arterial measurements as described above. Where the deep veins are in a healthy condition, the Doppler signal will be strongly damped indicating the closure of the venous valves and resultant lack of retrograde blood flow.

The invention may also be used to assess the condition of the long saphenous vein prior to its use in femorodistal bypass graft surgery. If the vein is in poor condition, this will be evident as a result of examination by the technique and with the apparatus of this invention.

Furthermore, the pulsatile wave form applied by the method of this invention to a body part may be used to generate a signal from a distal blood vessel which signal is then subjected to data processing so that the invention is used to generate a transmission line analysis for blood vessels located between the point of application of the pressure wave form and the point at which blood flow measurements are taken. In this embodiment, the pulsatile pressure wave form may have a higher frequency than that used in arterial and venous assessment.

In the embodiments described above, the inflatable cuff should be of a size adequate to compress muscle within the body part about which the cuff is located.

Where the pressure is derived from compressed air, the connections (e.g. tubing) between the compressed gas source and the inflatable cuff should be of wide bore in order to prevent undue pressure loss and/or pulse delays.

## Claims

1. A method of testing the vascular condition of a human or a non-human animal, the method being characterised by the steps of:
i) selecting a portion of the body of the animal which has restricted blood flow due to an ischaemia;
ii) applying a pulsatile pressure waveform as a series of pressure pulses to a first part of said portion in order to generate a pulsatile pressure waveform in the blood in said portion, and producing a first signal (3) representative of said applied pulsatile pressure waveform;
iii) non-invasively detecting the pulsatile pressure waveform in the blood at a second part of said portion spaced from said first part, and producing a second signal (5) representative of the detected pulsatile pressure waveform; and
iv) comparing the waveform of the first signal (3) with the waveform of the second signal (5).

2. A method according to claim 1, wherein said second part is distal from said first part.

3. A method according to claim 1 or 2, wherein the pulsatile pressure waveform in the blood at said second part is detected by means of Doppler ultrasonography.

4. A method according to claim 1, 2 or 3, wherein said pressure pulses are generated pneumatically.

5. A method according to claim 1, 2, 3 or 4, wherein said pressure pulses have a pulse rate in the range 0.3 - 1.5 pulses per second.

6. A method according to claim 5, wherein said pressure pulses have a pulse rate in the range 0.5 - 1.0 pulses per second.

7. A method according to any preceding claim, wherein said pressure pulses have a maximum pressure in the range (19-66.5) x 10³ Nm⁻²) (150-500 mm Hg) (gauge).

8. A method according to claim 7, wherein said pressure pulses have a maximum pressure in the range (26-40) x 10³ Nm⁻² (200-300 mm Hg) (gauge).

9. Non-invasive apparatus for use in testing the condition of blood vessels downstream from an ischaemia in a human or non-human animal, which apparatus is characterised in that there is provided:
i) means (1) for producing blood flow having a pulsatile waveform downstream of the ischaemia by applying to the periphery of a first part of the body of said animal a pulsatile pressure waveform, and for generating a first signal (3) representative of said applied pulsatile waveform;
ii) means (4) for detecting the waveform of the blood flow at a second part of the body of said animal distal to said first part, and for generating a second signal (5) representative of said detected waveform; and
iii) means enabling a comparison to be made between the waveform of said first signal (3) and the waveform of said second signal (5).

10. Apparatus as claimed in claim 9, wherein the pulsatile pressure waveform producing means comprise an inflatable cuff for application to the periphery of an area of the animal.

11. Apparatus as claimed in claim 10, wherein said inflatable cuff is a sphygmomanometer cuff.

12. Apparatus as claimed in claim 10 or 11, comprising a pressure control system in the form of a pressure transducer which is arranged to actuate a three-way valve, whereby in use compressed gas is admitted to the inflatable cuff until a predetermined pressure cut-off point is reached, whereupon the pressure transducer stops the supply of compressed gas and vents the interior of the inflatable cuff to the atmosphere.

## Patentansprüche

1. Verfahren zum Testen des Gefäßzustands bei Mensch oder Tier, gekennzeichnet durch die Schritte:
(i) Wählen eines Körperglieds des Wesens, das wegen einer Ischämie einen gedrosselten Blutdurchfluß besitzt;
(ii) Anlegen einer pulsartigen Druckwellenform - in Form einer Reihe von Druckimpulsen - an einen ersten Gliedbereich, um im Blut des Gliedes eine pulsartige Druckwellenform zu erzeugen, und zum Erzeugen eines ersten Sigals (3), das die angelegte pulsartige Druckwellenform beschreibt;
(iii) nicht-invasives Erfassen der pulsartigen Druckwellenform im Blut eines zweiten Gliedbereichs, der zum ersten Bereich beabstandet ist, und zum Erzeugen eines zweiten Signals (5), das die erfaßte pulsartige Druckwellenform beschreibt; und
(iv) Vergleichen der Wellenform des ersten Signals (3) mit der Wellenform des zweiten Signals (5).

2. Verfahren nach Anspruch 1, wobei der zweite Bereich distal zum ersten Bereich liegt.

3. Verfahren nach Anspruch 1 oder 2, wobei die pulsartige Druckwellenform im Blut des zweiten Bereichs durch ein Doppler-Ultraschallbild erfaßt wird.

4. Verfahren nach Anspruch 1, 2 oder 3, wobei die Druckimpulse pneumatisch erzeugt werden.

5. Verfahren nach Anspruch 1, 2, 3 oder 4, wobei die Druckimpulse eine Pulsrate im Bereich von 0,3 bis 1,5 Pulsen pro Sekunde haben.

6. Verfahren nach Anspruch 5, wobei die Druckimpulse eine Pulsrate im Bereich von 0,5 bis 1,0 Pulsen pro Sekunde haben.

7. Verfahren nach irgendeinem vorhergehenden Anspruch, wobei die Druckimpulse einen Höchstdruck im Bereich von 19 bis 66,5 x 10³ Nm⁻² (150 bis 500 mm Hg) haben.

8. Verfahren nach Anspruch 7, wobei die Druckimpulse einen Höchstdruck im Bereich von 26 bis 40 x 10³ Nm⁻² (200 bis 300 mm Hg) haben.

9. Nicht-invasives Gerät zum Testen des Zustands von Blutgefäßen stromab einer Ischämie in Mensch oder Tier, wobei das Gerät gekennzeichnet ist durch
(i) eine Einrichtung (1) zum Erzeugen stromab einer Ischämie eines Blutstroms mit einer pulsartigen Wellenform, indem eine pulsartigen Druckwellenform an die Peripherie eines ersten Körperglieds des Wesens angelegt wird, und zum Erzeugen eines ersten Signals (3), das für die angelegte Wellenform steht;
(ii) eine Einrichtung (4) zum Erfassen der Wellenform des Blutstroms in einem zweiten Bereich, der zum ersten Bereich des Körpers des Wesens distal liegt, und zum Erzeugen eines zweiten Signals (5), das die erfaßte Wellenform darstellt; und
(iii) eine Einrichtung, die einen Vergleich zwischen der Wellenform des ersten Signals (3) und der Wellenform des zweiten Signals (5) erlaubt.

10. Gerät nach Anspruch 9, wobei die Einrichtung zur Erzeugung einer pulsartigen Druckwellenform eine aufblasbare Manschette aufweist, die man an einen Peripheriebereich des Wesen anlegen kann.

11. Gerät nach Anspruch 10, wobei die aufblasbare Manschette eine Sphygmomanometer-Manschette ist.

12. Gerät nach Anspruch 10 oder 11, umfassend ein Drucksteuerungssystem in Form eines Druckübertragers, der ausgelegt ist, ein Dreiwegeventil zu betätigen, wodurch bei Gebrauch Gas, das unter Druck steht, in die aufblasbare Manschette gelassen wird, bis man einen vorbestimmten Druckendpunkt erreicht, worauf der Druckübertrager die Zufuhr von unter Druck stehendem Gas unterbricht und das Innere der aufblasbaren Manschette in die Umgebung ventiliert.

## Revendications

1. Un procédé d'examen des conditions vasculaires d'un être humain ou d'un animal le procédé étant caractérisé par les opérations consistant à :
i) choisir une partie du corps de l'être qui présente un débit de sang restreint dû à une ischémie ;
ii) appliquer une pression pulsée en forme d'onde sous la forme d'une série d'impulsions de pression à une première portion de ladite partie afin d'engendrer une pression pulsée en forme d'onde dans le sang de ladite partie et produire un premier signal (3) représentatif de ladite pression pulsée en forme d'onde appliquée ;
iii) détecter sans introduction la pression pulsée en forme d'onde dans le sang à une seconde portion de ladite partie espacée à partir de ladite première partie et produire un second signal (5) représentatif de la pression pulsée en forme d'onde détectée ; et
iv) comparer la forme d'onde du premier signal (3) à la forme d'onde du second signal (5).

2. Un procédé suivant la revendication 1, dans lequel la seconde partie est distale de ladite première partie.

3. Un procédé suivant la revendication 1 ou 2, dans lequel la pression pulsée en forme d'onde dans le sang à la seconde partie est détectée au moyen d'une ultrasonographie Doppler.

4. Un procédé suivant la revendication 1, 2 ou 3, dans lequel lesdites impulsions de pression sont engendrées pneumatiquement.

5. Un procédé suivant la revendication 1, 2, 3 ou 4, dans lequel les impulsions de pression ont une cadence d'impulsions de 0,3 à 1,5 impulsions par seconde.

6. Un procédé suivant la revendication 5, dans lequel les impulsions de pression ont une cadence d'impulsions de 0,5 à 1 impulsion par seconde.

7. Un procédé suivant l'une quelconque des revendications précédentes, dans lequel lesdites impulsions de pression présentent une pression maximum comprise entre (19-66,5) x 10³ Nm⁻² (150-500 mm de Hg) (jauge).

8. Un procédé suivant la revendication 7, dans lequel lesdites impulsions de pression présentent une pression maximum comprise entre (26-40) x 10³ Nm⁻² (200-300 mm de Hg) (jauge).

9. Appareil sans introduction pour être utilisé dans l'examen de la condition de vaisseaux sanguins en aval d'une ischémie dans un être humain ou un animal, appareil qui est caractérisé en ce qu'il comprend :
i) des moyens (1) pour produire un écoulement sanguin présentant une forme d'onde pulsée en aval de l'ischémie, en appliquant, à la périphérie de la première partie du corps dudit être, une pression pulsée en forme d'onde et pour créer un premier signal (3) représentatif de ladite pression pulsée en forme d'onde ;
ii) des moyens (4) pour détecter la forme d'onde de l'écoulement sanguin à une seconde partie du corps dudit être, distale de ladite première partie et pour produire un second signal (5) représentatif de ladite forme d'onde détectée ; et
iii) des moyens permettant d'effectuer une comparaison entre la forme d'onde dudit premier signal (3) et la forme d'onde dudit second signal (5).

10. Appareil comme revendiqué à la revendication 9, dans lequel les moyens de production de la pression pulsée en forme d'onde comprennent une manchette gonflable destinée à être appliquée à la périphérie d'une zone de l'être.

11. Appareil comme revendiqué à la revendication 10, dans lequel ladite mancheite gonflable est une manchette sphygmomanométrique.

12. Appareil comme revendiqué à la revendication 10 ou 11, comprenant un système de commande de pression sous forme d'un transducteur de pression qui est disposé pour actionner une value trois voies de sorte qu'en utilisation, le gaz comprimé est dirigé à la manchette gonflable jusqu'à ce qu'une pression de coupure prédéterminée soit atteinte en dépendance de quoi le transducteur de pression arrête l'alimentation de gaz comprimé et met l'intérieur de la manchette gonflable à l'atmosphère.
